# EUROPÄISCHE PATENTSCHRIFT

(11) **EP 4 472 692 B1**
(45) Veröffentlichungstag und Bekanntmachung des Hinweises auf die Patenterteilung: **10.12.2025**
(21) Anmeldenummer: 23731948.8
(22) Anmeldetag: 02.06.2023
(51) Int. Cl.: A61M 1/02

(54) **PREDONATIONBEUTELSYSTEM ENTHALTEND EIN ANTIKOAGULANS UND BLUTBEUTELSYSTEM UMFASSEND DAS PREDONATIONBEUTELSYSTEM**
PREDONATION BAG SYSTEM CONTAINING AN ANTICOAGULANT, AND BLOOD BAG SYSTEM COMPRISING THE PREDONATION BAG SYSTEM
SYSTÈME DE POCHE DE PRÉ-TRANSFUSION CONTENANT UN ANTICOAGULANT, ET SYSTÈME DE POCHE DE SANG COMPRENANT LE SYSTÈME DE POCHE DE PRÉ-TRANSFUSION

(30) Priorität: 03.06.2022 DE 102022114101
(43) Veröffentlichungstag der Anmeldung: 11.12.2024
(73) Patentinhaber: Blutspendedienst der Landesverbände des Deutschen Roten Kreuzes Niedersachsen, Sachsen-Anhalt, Thüringen, Oldenburg und Bremen g.G.m.b.H., 31832 Springe (DE)
(72) Erfinder: SCHILLING, Jana, 30171 Hannover (DE); LACH, Marcus, 31860 Emmerthal (DE)
(74) Vertreter: Müller Schupfner & Partner Patent- und Rechtsanwaltspartnerschaft mbB (Hamburg)
(86) Internationale Anmeldenummer: PCT/DE2023/100420
(87) Internationale Veröffentlichungsnummer: WO 2023/232198

(56) Entgegenhaltungen:
- CA-A1- 2 484 628
- CN-U- 201 631 679
- DE-A1- 3 030 020
- DE-U1- 202013 011 482
- US-A1- 2006 212 020

## Beschreibung

Die Erfindung betrifft ein Blutbeutelsystem umfassend ein Predonationbeutelsystem mit einem Predonationbeutel zur Befüllung mit Vollblut während der Blutentnahme, wobei dem Vollblut im Predonationbeutel ein Antikoagulans zugefügt ist, so dass die Abfüllung des Vollblutes aus dem Predonationbeutel in Probenbehälter für die Blutanalytik erst im Labor stattfinden kann und nicht am Ort der Blutspende.. Weiterhin wird ein entsprechendes Verfahren zur Blutentnahme mittels des Blutbeutelsystems und zur Analytik des Vollblutes eines jeden Spenders vorgeschlagen.

### EINFÜHRUNG UND STAND DER TECHNIK

Blutprodukte werden von Krankenhäusern, Blutbanken und anderen medizinischen Einrichtungen für die Verwendung bei Bluttransfusionen bevorratet. Aus einer Vollblutspende eines Spenders werden unterschiedliche Blutprodukte gewonnen, um einem Patienten die Blutbestandteile zu verabreichen, die dieser benötigt. Durch die Auftrennung wird zum einen das Risiko von Nebenwirkungen und Unverträglichkeiten stark gesenkt und zum anderen können durch die Auftrennung des Blutes in seine Bestandteile für jeden dieser Bestandteile optimale Bedingungen im Hinblick auf Haltbarkeit, Lagerfähigkeit und Wirksamkeit erreicht werden.

Im Rahmen einer Blutspende wird zunächst Vollblut von einzelnen Spendern gesammelt. Bei der Blutspende wird das Blut eines jeden Spenders, das sogenannte Vollblut, in ein geschlossenes Blutbeutelsystem geleitet. Das Blutbeutelsystem ist so konstruiert, dass das Vollblut im Vollblutbeutel (häufig auch als Sammelbeutel bezeichnet) zunächst gesammelt wird und sodann Aufbereitungs- bzw. Separationsbehandlungen erfährt und die so gewonnenen einzelnen Blutkomponenten in separate, verbundene Beutel (Transferbeutel) überführt werden. Hierbeiwird das Blut von einem Spender in seine Bestandteile aufgetrennt. Die Auftrennung erfolgt durch Zentrifugieren. Beim Zentrifugieren wird eine obere Plasmaschicht, ggf. eine dünnere Zwischenschicht, die als "buffy coat layer" bezeichnet wird und die Leukozyten und Thrombozyten enthält, und eine untere Schicht von Erythrozyten erhalten.

Es ist erforderlich die Leukozyten abzutrennen. Dies erfolgt durch Filtration vor oder nach dem Schritt der Auftrennung durch Zentrifugieren. Weiterhin kann das Vollblut oder die einzelnen Blutprodukte einem Schritt der Virusinaktivierung unterzogen werden. Ein Blutbeutelsystem ist in vielen Anwendungen (wie auch für die vorliegende Erfindung) so gestaltet, dass ein Blutbeutelsystem genau nur einem Spender zuzuordnen ist.

Obiges Verfahren erfordert die sorgfältige Kennzeichnung der Probenbehälter damit diese eindeutig einem Spender und dem dazugehörigen Blutbeutelsystem zuordbar sind.

Es ist eine große Anzahl unterschiedlicher Blutbeutelsysteme zum Sammeln, Trennen und Lagern der Blutkomponenten bekannt. In der Regel wird zum Sammeln von Vollblut und Auftrennen in die Blutkomponenten ein geschlossenes steriles Blutbeutelsystem verwendet, wie z.B. vom Fenwal-Typ. Dieses System enthält einen relativ großen Vollblutbeutel zum Sammeln einer Blutspende, einen Filter und einen oder mehrere Transferbeutel, die durch Verbindungsschläuche an den Vollblutbeutel angeschlossen sind.

Den unterschiedlichen Blutbeutelsystemen ist in der Regel gemeinsam, dass das Vollblut ohne Verwendung externer Pumpen oder ähnlicher Vorrichtungen vom Spender in den Vollblutbeutel fließt. Hierzu umfasst die Blutentnahme das Einführen eines Venenzugangsgeräts, z. B. einer Venenpunktionsnadel, in die Vene des Spenders und die Entnahme von Blut aus dem Spender durch dasVenenzugangsgerät. Die Nadel ist über einen Adapter mit einem Schlauch verbunden, durch den das Vollblut in den Vollblutbeutel gelangt. Venenzugangsgerät wie eine Venenpunktionsnadel, Schläuche und Beutel bilden das Blutbeutelsystem, das vorsterilisiert und nach einmaligem Gebrauch entsorgtwird.

Der Vollblutbeutel enthält ein Antikoagulans, da Blut nach einiger Zeit anfängt zu gerinnen und an den Wänden der Kunststoffoberflächen, mit denen es in Kontakt kommt, zu haften oder sonst wie zu koagulieren. Die verwendet Antikoagulanzien müssen im europäischen Arzneibuch hinterlegt sein. Es handelt sich typischerweise um CPD (Citrat-Phosphat-Dextrose) oder ACD (Acid-Citrat-Dextrose).

Eine wichtige Überlegung bei jeder Blutentnahmetechnik oder jedem Blutbeutelsystem ist sicherzustellen, dass das Blutbeutelsystem nicht durch in der Luft befindliche Bakterien oder andere Fremdstoffe kontaminiert wird, die die Sterilität des Systems beeinträchtigen könnten. Somit wird die Sterilität des oben beschriebenen Blutbeutelsystems nach dem Stand der Technikgewährleistet, indem die Strömungswege und das Innere der Beutel nicht bzw. auf ein Minimum beschränkt der äußeren Umgebung ausgesetzt werden. Solche Systeme werden allgemein als "geschlossene Systeme" bezeichnet.

Bei jeder Blutspende wird das Vollblut eines Spenders auf die Blutgruppe und das Vorhandensein von Krankheitserregern wie Viren und Bakterien im Blut des Spenders getestet. Typischerweise erfordert das Testen des gesammelten Blutes die Entnahme einer oder mehrerer Proben je Spender zum oder nahe dem Zeitpunkt der Sammlung. Das Entnehmen von Proben aus dem Vollblutbeutel unter Aussetzen des gesammelten Blutes in dem Vollblutbeutel der äußeren Umgebung ist jedoch unerwünscht. Häufig stört auch das Antikoagulans im Vollblutbeutel die Analytik.

Bei der Vollblutspende findet in der Regel neben der Blutabnahme vor Ort während der Spende zeitgleich die Befüllung der Probenbehälter statt, welche für Analysen im Labor notwendig sind. In den Probenbehälter befindet sich ein Antikoagulans, damit die Proteine im Blut bis zur Überprüfung im Labor nicht ausfallen. Bei dem Antikoagulans kann es sich z.B. um Na-Citrat, CPD (Citrate-Phosphate-Dextrose), CP2D (Citrate Phosphate-Double-Dextrose), CPDA (Citrate-Phosphate-Dextrose-Adenine) oder ACD ("Acid-Citrate-Dextrose") handeln. Probenbehälter werden z.B. in der WO 2003/095974 A2 vorgeschlagen.

Nach einem Beispiel wird Blut zur Probenahme in einem vergrößerten Abschnitt der Zuleitung zum Vollblutbeutel gesammelt. Sobald das gewünschte Blutvolumen für die Probenahme im vergrößerten Schlauchabschnitt gesammelt wurde, wird die Zuleitung zum Vollblutbeutel zwischen Vollblutbeutel und dem vergrößerten Abschnitt geschlossen, die Nadel vom Spender entfernt und das Blut aus dem Schlauchabschnitt wird in Probenbehälter überführt. Ein solches Blutbeutelsystem hat sich als weniger geeignet für die vorliegende Anwendung erwiesen.

Ein Blutbeutelsystem für Nabelschnurblut ist beispielsweise in der DE 202013011482 U1 zur Entnahme von Nabelschnurblut vorgeschlagen worden. Hier dient der Vollblutbeutel als Einfrierbeutel und der vergrößerte Schlauchabschnitt als Reservoirbereich. Weitere Blutbeutelsysteme sind aus der CN 201631679 U oder der DE 3030020 A1 bekannt, aber ohne Predonationsbeutel. Die CA2484628 A1 und die US2006/212020 A1 betreffen Probennahmegefäße u.a. für Vollblut unter Verwendung von EDTA als Antikoagulans.

### AUFGABE DER ERFINDUNG

Obwohl die bekannten Techniken im Allgemeinen zufriedenstellend funktionieren, werden weiterhin Anstrengungen unternommen, um Verbesserungen auf dem Gebiet der Blutentnahme und Gewinnung von Blutprodukten bereitzustellen, insbesondere was Blutbeutelsysteme anbetrifft, die einen höheren Grad der Automatisierung erlauben. Vorliegend ist es Aufgabe der Erfindung die Analytik des Vollbluts zu vereinfachen und zu automatisieren und die Probenahme zu vereinfachen bzw. zu systematisieren.

### ZUSAMMENFASSUNG DER ERFINDUNG

Diese und weitere Aufgaben werden durch den Gegenstand der unabhängigen Patentansprüche gelöst. Bevorzugte Ausführungsformen sind Gegenstand der Unteransprüche oder nachfolgend beschrieben.

Gegenstand der vorliegenden Erfindung ist ein Blutbeutelsystem umfassend ein Predonationbeutelsystem, das die Entnahme von Blut aus einem Predonationsbeutel als Teil des Blutbeutelsystems in Probenbehälter nicht mehr lokal am Ort der Blutabnahme, sondern zentral und vorzugsweise automatisiert in einem Labor bei oder während derBlutverarbeitung ermöglicht.

Das Predonationbeutelsystem umfasst zumindest einen Predonationbeutel, einen Venenpunktionsadapter und eine Blutentnahmeleitung mit
a) einem gegenüber der Blutentnahmeleitung verschließbaren ersten Schlauchabschnitt als Zufuhr von der Blutentnahmeleitung in den Predonationbeutel und
b) einen zweiten Schlauchabschnitt als Zufuhr von der Blutentnahmeleitung in weitere Teile des Blutbeutelsystems, z. B. in einen Vollblutbeutel (wobei der Vollblutbeutel nicht Teil des Predonationbeutelsystems ist), insbesondere unmittelbar in einen Vollblutbeutel,
wobei der Predonationbeutel oder der erste Schlauchabschnitt oder beideein Antikoagulans für Vollblut umfasst/umfassen und das Antikoagulans EDTA ist. Insbesondere ist das Antikoagulans K2-EDTA (Di-Kalium EDTA) und/oder K3-EDTA (Tri-Kalium EDTA). Der Probenbehälter muss dann kein Antikoagulans mehr enthalten.

Von der Blutentnahmeleitung zweigt z.B. der erste Schlauchabschnitt und der zweite Schlauchabschnitt ab. Der zweite Schlauchabschnitt kann auch dieselbe Leitung wie die Blutentnahmeleitung sein, die nach der Abzweigung des ersten Schlauchabschnitts dann zweiter Schlauchabschnitt heißt und umgekehrt kann der erste Schlauchabschnitt auch dieselbe Leitung wie die Blutentnahmeleitung sein, die nach der Abzweigung des zweiten Schlauchabschnitts dann erster Schlauchabschnitt heißt.

Der Vorteil einer zentralen Abfüllung der Probenbehälter liegt u.a. in der Steuerung der Prozessschritte. Es sind viele Spenderinformationen in der Datenbank des Blutspendedienstleisters hinterlegt, die abgerufen werden können, so dass eine Entscheidung über die Anzahl und Art der notwenigen Tests und damitder zu befüllenden Probenbehälter erst nach Abgleich mit den vorhandenen Daten gemacht werden kann. Ein weiterer Vorteil ist das zentrale, automatisierte Abschweißen der Predonationbeutel. Das Risiko einer Belüftung des Beutels ist hiermit deutlich geringer als wenn das Abschweißen oder sonstige Abtrennen am Ort der Blutspende erfolgt.

Für den Transport und die spätere Befüllung der Probenbehälter wird das Vollblut im Predonationsbeutel des Blutbeutelsystems, aus dem später der oder die Probenbehälter befüllt werden, mit einem geeigneten Antikoagulans versehen. Dieses verhindert die Gerinnung des Bluts. Als geeignetes Antikoagulans für das Vollblut im Predonationsbeutel hat sich überraschend EDTA erwiesen, insbesondere K2-EDTA und/oder K3-EDTA. Sowohl im Infektions- und Blutgruppenserologischem Labor als auch bei der PCR Analyse und der molekularen Blutgruppenbestimmung kann K2- oder K3- EDTA eingesetzt werden. Diese Labore machen eine Vielzahl von Tests (z.B. >10), die alle auf das gleiche Antikoagulanz umgestellt werden müssen.

Ein weiteres Kennzeichen der erfindungsgemäßen Blutbeutelsysteme mit Predonationsbeutelsystem ist, dass das Antikoagulans im Vollblut des Predonationsbeutel ein anderes ist als im Vollblutbeutel. Dies erfordert, dass das Vollblut im Predonationsbeutel sogesammelt werden kann, dass weder das Antikoagulans für den Predonationsbeutel noch das Vollblut in dem das Antikoagulans gelöst ist, mit dem Spender bzw. Spenderblut oder dem Vollblut im Vollblutbeutel in Kontakt tritt, insbesondere ein Kontakt oder ein Vermischen mit dem Vollblut im Vollblutbeutel ist zu vermeiden. Im Vollblutbeutel kann dann beispielsweise als Antikoagulans CPD, CPDA1 und/oder CP2D eingesetzt werden.

Die Erfindung betrifft weiterhin ein Verfahren zur Entnahme von Vollblut mittels des Blutbeutelsystems, wobei das mit Antikoagulans versehene Vollblut oder ein Teil davon aus dem Predonationbeutel nach Transport des Predonationbeutels in ein Labor in eine oder mehrere Probenbehälter gefüllt wird, vorzugsweise automatisiert, und der Probenbehälter einer Analyse zugeführt wird oder die Probenbehälter jeweils unterschiedlichen Analysen zugeführt werden.

Die Anzahl der Probenbehälter pro Predonationbeutel kann in Abhängigkeit von dem Spender und einem Abgleich mit einem Eintrag in einer Datenbank für den Spender bestimmt werden. Z.B. kann der Datenbank entnommen werden, welche Art der Analytik für den jeweiligen Spender notwendig ist und wie viele Probenbehälter hierfür erforderlich sind. Jedem Spender ist ein Predonationbeutel bzw. Blutbeutelsystem zugeordnet. Die Automatisierung erlaubt die richtige Anzahl von Probenbehälter auszuwählen, unnötige Probenbehälter zu vermeiden, und die Probenbehälter auf gleiche Tests oder Abfolgen von gleichen Tests für die unterschiedlichen Spender zusammenzustellen. Beispielsweise werden Erstspender einer größeren Anzahl von Tests unterzogen und es werden dementsprechend mehr Probenbehälter benötigt. Weiterhin wird der Arbeitsschritt der Befüllung und Kennzeichnung der Probenbehälter am Ort der Blutspende vermieden.

### DETAILLIERTE DARSTELLUNG DER ERFINDUNG

Nach der vorliegenden Erfindung erfolgt die Probenahme von Vollblut nicht aus dem Vollblutbeutel selbst, sondern der Strömungsweg des Vollblutes wird vor dem Vollblutbeutel abgeklemmt und das dem Spender entnommene Vollblut wird zunächst in den Probenbeutel (Predonationbeutel), z.B. über eine T- oder Y-Abzweigung, umgeleitet bis der Strömungsweg zum Predonationbeutel geschlossen wird, insbesondere durch einen zunächst geöffneten und später geschlossenen Verschluss im Bereich des ersten Schlauchabschnitts, und der Strömungsweg zum Vollblutbeutel geöffnet wird, insbesondere durch einen zunächst geschlossenen und später geöffneten Verschluss im Bereich des zweiten Schlauchabschnitts, um den Vollblutbeutel zu füllen.

Nach dieser Ausführungsform wird ein Einweg-Schlauchset als Teil des Blutbeutelsystems eingesetzt, das auf dem Hauptströmungsweg von der Blutentnahmeleitung in den zweiten Schlauchabschnitt mit dem ersten Schlauchabschnitt als "Dead-End"/ Sackgasse aufgesetzt ist und den Predonationbeutel, einen ersten Schlauchabschnitt und einen Adapter für den Probenbehälter als Probenahmestelle aufweist. Blut aus dem Predonationbeutel kann dann z.B. entnommen werden, indem ein Deckel am Adapter geöffnet wird, ein Probenbehälter, z.B. in Form eines Probenröhrchens, in den Adapter eingeführt wird und eine Nadel im Adapter die Membran des Probenbehältnisses durchsticht. Sodann fließt Vollblut über die Probenahmestelle in den aufgesetzten Probenbehälter, der z.B. unter Unterdruck steht.

Nach einer Ausführungsform der Erfindung ist das Innenvolumen des Predonationsbeutels 10 bis 50 mL groß, insbesondere 25 bis 35 mL oder enthält das zuvor angegebene Volumen an Vollblut. Insbesondere beträgt die Konzentration an Antikoagulans im Vollblut 1,2 bis 2 mg/mL, bevorzugt 1,4 bis 1,8 mg/mL, wie z.B. etwa 1,6 mg/mL. Durch das Antikoagulans im Predonationsbeutel wird gewährleistet, dass die gewünschten Tests mit dem Vollblut in dem oder den Probenbehältern zeitversetzt im Labor durchgeführt werden können. Das erfindungsgemäß eingesetzte Antikoagulans im Predonationsbeutel stört nicht die Laboranalytik und verhält sich somit in den Tests neutral.

Das Antikoagulans kann im Predonationbeutel oder im ersten Schlauchabschnitt, vorzugsweise jenseits des Verschlusses im ersten Schlauchabschnitt und zum Predonationbeutel hin, vorliegen:
1. als Flüssigkeit, z.B. gelöst in Wasser,
2. als Beschichtung, d.h. immobilisiert als Teil einer Innenwandbeschichtung,
3. als Feststoff (z.B. als Tablette), und/oder
4. als Feststoff oder Flüssigkeit in einer Kapsel, die durch äußeren Druck oder durch Lösen öffnet.

Das Antikoagulans kann beispielsweise
a) als Feststoff auf die innere Oberfläche des ersten Schlauchabschnitts oder eines Röhrchens, das in den ersten Schlauchabschnitt eingesetzt ist, aufgebracht sein und/oder
b) als Feststoff auf die innere Oberfläche des Predonationbeutels aufgebracht sein.

Nach einer Ausgestaltung der Erfindung wird das Antikoagulans in Wasser gelöst in den Predonationbeutel eingebracht. Damit ist sichergestellt, dass sich das **EDTA** im Vollblut einfach verteilt, wenn das Vollblut z.B. durch den Druck in der Blutbahn des Spenders in den Predonationbeutel einströmt.

Das Antikoagulans kann auch auf die Beutelinnenwand des Predonationbeutels und/oder in einem Schlauchabschnitt oder einem in den Schlauchabschnitt eingesetztem Röhrchen zum Predonationbeutels hin nach der Absperrmöglichkeit (Verschluss) immobilisiert werden. Durch Kontakt von dem Vollblut mit dem immobilisierten **EDTA** löst sich dieses auf. Die Beschichtung der Innenwände erfolgt z.B. durch Sprühtrocknen.

Weiterhin besteht die Möglichkeit, dass sich eine geeignete Menge Antikoagulans in fester Form im Predonationsbeutel befindet und sich das Antikoagulans im Vollblut löst, sobald es in den Predonationsbeutel hineinfließt und mit diesem in Kontakt tritt, ggf. unterstützt durch Schütteln des Predonationsbeutels.

Auch kann eine Tablette, z.B. in einer Pillenkammer, in das Schlauchstück (erster Schlauchabschnitt) zum Predonationbeutel eingebracht werden und löst sich durch das Vorbeifließen des Vollbluts und ggf. anschließendes Schütteln (manuell oder auf der Mischwaage) auf. Die Tablette kann auch einfach nur in den Predonationbeutel eingebracht werden.

Nach einer anderen Ausgestaltung ist das Antikoagulans in eine Kapsel eingebracht, deren Hülle sich im Kontakt mit Vollblut auflöst oder die mechanisch aufgebrochen werden muss, damit das Antikoagulans im Predonationbeutel oder in einer Zuleitung in diesen freigesetzt wird. Z.B. wird die Kapsel aufgebrochen, wenn die Zuleitung zum Predonationbeutel hinter dem Ort der Kapsel verschlossen wird. Ein Rückfluss zum Spender oder in Richtung des Vollblutbeutels ist zu verhindern.

Der erste Schlauchabschnitt weist insbesondere einen Verschluss (vorzugsweise zunächst offen und später verschließbar) auf. Das Antikoagulans ist vorzugsweise ausschließlich im ersten Schlauchabschnitt hinter dem Verschluss und/oder im Predonationbeutel (ebenfalls hinter dem Verschluss im ersten Schlauchabschnitt) angeordnet.

Durch ein Ventil oder eine Absperrung in Form des verschließbaren und nur ggf. wieder zu öffnenden mechanischen Verschlusses in der Zuleitung zum Predonationbeutel ist dafür gesorgt, dass das EDTA bzw. Vollblut mit darin enthaltenem EDTA nicht zurückfließen kann. So wird wirkungsvoll verhindert, dass das Antikoagulans mit dem Spender und/oder dem Vollblut im Vollblutbeutel in Kontakttritt.

Nach einer Ausgestaltung ist der Verschluss im ersten Schlauchabschnitt zunächst in der Grundstellung geschlossen, lässt sich dann öffnen und schließt beim zweiten Schließen, insbesondere irreversibel, lässt sich also nicht ein zweites Mal öffnen. Oder der Verschluss ist zunächst in der Grundstellung geöffnet und schließt beim ersten Schließen, insbesondere irreversibel, und lässt sich dann nicht ein zweites Mal öffnen.

Der zweite Schlauchabschnitt kann als Verschluss beispielsweise ein Abbrechteil mit Sollbruchstelle im Inneren des zweiten Schlauchabschnitts aufweisen, das mechanisch abgebrochen wird und dadurch den Durchfluss in den Vollblutbeute über den zweiten Schlauchabschnitt freigibt. Ebenso, aber weniger bevorzugt, kann das Abbrechteil im ersten Schlauchabschnitt angeordnet sein. Ein Beispiel für eine mögliche Ausgestaltung des Abbrechteils als einen irreversibel zu öffnenden Verschluss ist aus der DE 3030020 A1 ersichtlich.

Die zentrale Probenbehälterabfüllung lässt sich auf verschiedene Arten realisieren. Nach einer Ausführungsform wird der Predonationbeutel vom restlichen Blutbeutelsystem durch steriles Schweißen abgetrennt (insbesondere zwischen Verschluss des ersten Schlauchabschnitts und Predonationsbeutel), wobei beide Enden durch Verschweißen verschlossen werden oder die Zuleitung ist vom Predonationbeutel aus betrachtet hinter dem Verschluss abtrennbar. Das Verschweißen erfolgt beispielsweise durch bereichsweises thermisches Erhitzen und bereichsweises Zusammenpressen des Schlauchabschnitts und eine Trennung z.B. durch Stanzen in der Verschweißzone oder das Ausbilden eine Abreißlinie als Materialschwächung in der Verschweißzone. Auf diese Weise werden durch das Verschweißen beide Schlauchenden verschlossen.

Im Anschluss werden die Probenbehälter zentral im Labor mit Blut aus dem Predonationsbeutel befüllt. Dieser Schritt kann automatisiert durchgeführt werden. Hierfür wird zunächst die spezifische Präparatenummer bzw. Identifikation des Predonationbeutels ausgelesen und die gewünschte Anzahl an Probenbehälter bestimmt, wobei die Probenbehälter der einzelnen Spende stets eindeutig zugeordnet werden können, indemz.B. jeder Probenbehälter mit einer Identifikation versehen ist, die eineZuordnung zu dem Spender erlaubt. Dies kann durch Etikettieren erfolgen oder durch Zuordnung einer Probenidentifikation und/ oder einer Spenderidentifikation in einer Datenbank. Danach wird das mit EDTA versetzte Vollblut aus dem Predonationbeutel in den Probenbehälter überführt. Eine Möglichkeit der Befüllung der Probenbehälter ist die Nutzung eines Adapters, wie er weiter oben beschrieben ist und auch für die manuelle Probenbehälterbefüllung verwendet werden kann. Es ist aber ebenfalls möglich, dass die Befüllung direkt über einen Schlauch oder eine Kanüle realisiert wird. Mit der Kanüle kann in den Predonationsbeutel gestochen werden, um eine Probe zu entnehmen. Kontaminationen mit Fremdblut sind zu vermeiden.

Wenn gewünscht, kann das Predonationsbeutelsystem auch vom Rest des Blutbeutelsystems abgeschweißt werden, insbesondere zwischen Klemme am zweiten Schlauchabschnitt und Vollblutbeutel, indem durch steriles Schweißen (z.B. wie oben beschrieben) der zweite Schlauchabschnitt beidseitig verschlossen wird und in der Schweißstelle eine Trennung z.B. durch Stanzen herbeigeführt wird oder eine Abreißlinie als Materialschwächung vorgeprägt wird.

Die Erfindung wird anhand der nachfolgenden Figuren erläutert, ohne auf diese beschränkt zu sein. Es zeigen:
Fig.1 das Predonationbeutelsystem für das Blutbeutelsystem und
Fig. 2 ein Blutbeutelsystem mit Predonationbeutelsystem.

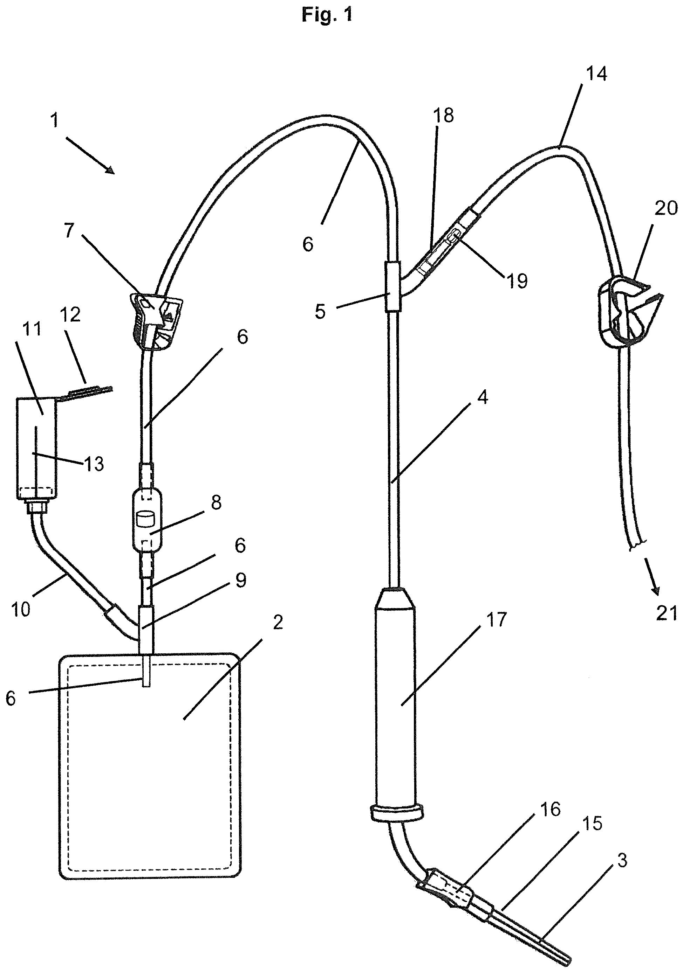

Fig. 1 zeigt eine Darstellung der Predonationbeutelsystems 1. Das Predonationbeutelsystem 1 umfasst einen Predonationbeutel 2, eine Venenpunktionsnadel 3, einen Blutentnahmeschlauch 4 von der Venenpunktionsnadel 3 zu einer ersten Y- Verbindung 5, von welcher sich der Strom aus dem Blutentnahmeschlauch 4 in einen ersten Schlauchabschnitt 6 fortführt, der in dem Predonationbeutel 2 endet.

Der andere Arm der Y-Verbindung 5 führt in einem zweiten Schlauchabschnitt 14 der zum Vollblutbeutel 21 führt.

An dem freien Ende der Blutentnahmeleitung 4 ist über einen Venenpunktionsadapter 16 die Venenpunktionsnadel 3 angeschlossen, die zurEntnahme des Vollbluts von dem Spender dient. Die Venenpunktionsnadel 3 ist von einer aufsteckbaren konisch zulaufenden Kappe 15 umschlossen. Weiterhin ist eine Schutzglocke 17 beweglich entlang des Blutentnahmeschlauches 4 verfahrbar und rastet am Ende Blutentnahmeschlauches auf dem Adapter ein, damit die Schutzglocke 16 die Venenpunktionsnadel 3 und die Kappe 15 schützend umspannt und in ihrem Inneren aufnimmt.

Von der Blutentnahmeleitung 4 zweigt am anderen Arm der Y-Verbindung 5 der zweite Schlauchabschnitt 14 ab. Teil der Y- Verbindung 5 ist ein in diesen Arm der Y-Verbindung eingesetztes Abbrechteil 18, das den Durchfluss durch den Arm der Y-Verbindung 5 versperrt. Das Abbrechteil 18 weist eine hohle Hülse und am Ende eine Olive 19 auf, die die Hülse in Flussrichtung verschließt. Die Hülse legt sich am anderen Ende mit einem Kragen dichtend in dem Arm des Y-Verbindung fest. Durch Umbiegen des Endes des Armes kann die Olive entlang einerSollbruchstelle abgebrochen werden, womit der Strom von Vollblut in den zweiten Schlauchabschnitt 14 durch die Hülse freigegeben wird.

Die Olive 19 verbleibt am Ende des Armes des Y-Stückes, weil deren äußerer Umfang Kämme aufweist, die verhindern, dass die Olive 19 in den zweiten Schlauchabschnitt 14 geschwemmt werden kann, andererseits aber das Blut u.a. durch Kammrillen in der Olive passieren lässt. Das Abbrechteil 18 ist eine Option und kann in gleicher Weise auch in den ersten Schlauchabschnitt 6 bzw. den Arm der Y- Verbindung 5 eingesetzt sein, der in den ersten Schlauchabschnitt 6führt.

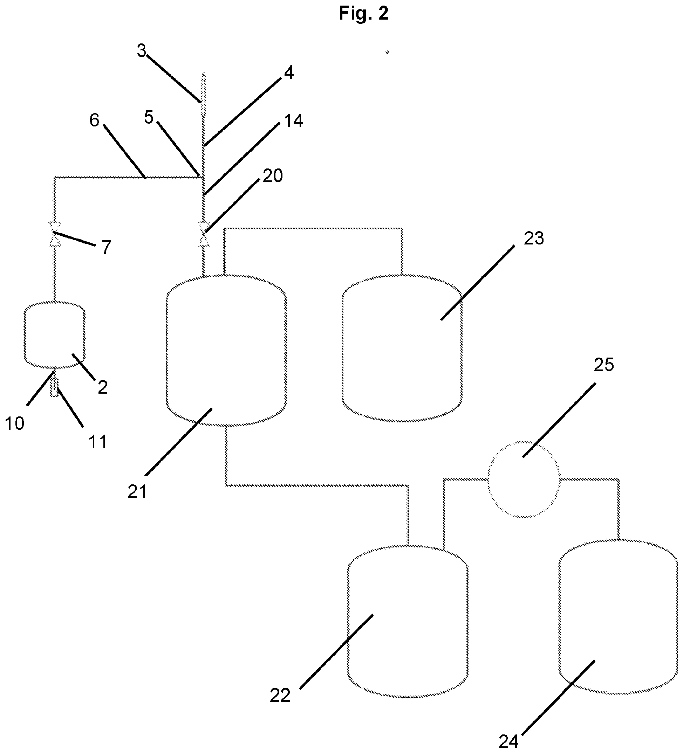

Darüber hinaus ist der zweite Schlauchabschnitt 14 mit einer zunächst offenen und später wieder verschließbaren Schlauchklemme 20 versehen, die verschlossen wird, nachdem der Vollblutbeutel 21 (siehe Fig. 2) ausreichend gefüllt ist und verhindern soll, dass Vollblut zurückfließt.

In den erste Schlauchabschnitt 6 ist EDTA in Form einer Tablette in die Pillenkammer 8 eingebracht. Durch Vorbeiströmen löst sich die Tablette auf und gibt das EDTA an das Vollblut ab, so dass schlussendlich das Vollblut in dem Predonationbeutel 2 mittels des Antikoagulants EDTA stabilisiert ist.

Über eine zweite Y-Verbindung 9 steht ist ein Probenahmeadapter 11 über einen Probennahmeschlauchabschnitt 10 in Fließverbindung mit dem Predonationbeutel 2 über einen Streckenabschnitt der ersten Schlauchabschnitts. Wird der Deckel 12 des Probennahmeadapters 11 geöffnet, kann ein Probenbehälter mit dem Septum in den zylindrischen Hohlraum des Probennahmeadapters 11 eingeführt werden. Aufgrund des Unterdruckes indem Probenbehälter füllt sich der Probenbehälter jeweils mit dem Vollblut enthaltend das Antikoagulans. In gleicher Weise können weitere Probengehälter, z.B. in Form von Probennahmeröhrchen, in einem Labor, d.h. nicht am Ort der Blutentnahme, automatisiert etikettiert und befüllt werden. Diese werden dann gesammelt und den entsprechenden Test in einer Mehrzahl zugeführt. Die Arme der Y-Verbindungen sind jeweils Teil der entsprechenden Schlauchabschnitte.

In Fig. 2 ist schematisch ein Blutbeutelsystem umfassend das Predonationbeutelsystem 1 der Fig. 1 dargestellt (allerdings ist hier der Probenahmeadapter 11 direkt über den Probennahmeschlauchabschnitt 10 mit dem Predonationbeutel 2 ohne zweite Y-Verbindung verbunden).

Über eine Venenpunktionsnadel 3 wird bei der Blutentnahme das Vollblut des Spenders in das Blutbeutelsystem geleitet, wobei ein erster Schlauchabschnitt 6 nach der Y-Verbindung 5 das Vollblut des Spenders zum Predonationbeutel 2 und ein zweiter Schlauchabschnitt 14 zum Vollblutbeutel 21 leitet. Zunächst wird das über die Venenpunktionsnadel3 entnommene Vollblut über den Blutentnahmeschlauch 4 und die Y-Verbindung 5 in den ersten Schlauchabschnitt 6 über eine irreversible Klemme 7 in den Predonationbeutel 2 geleitet. "Irreversible Klemme" 7 meint, dass diese entweder im Grundzustand geöffnet ist (bevorzugt) oder im Grundzustand geschlossen ist und sich öffnen lässt aber für beider Konstellationen sich nach dem ersten Schließen nicht mehr öffnen lässt. Der Predonationbeutel 2 ist über einen Probennahmeschlauchabschnitt 10 mit einem Adapter 11 verbunden, der zur Befüllung von Probenbehältern vorgesehen ist. Der Adapter 11 kann geöffnet werden, wobei eine zylinderförmige Führung freigelegt wird und weist in seinem Inneren eine Nadel auf für das Durchstoßen eines Septums auf. Sobald der Predonationbeutel 2 ausreichend befüllt ist, wird die irreversible Klemme 7 geschlossen und die reversible Klemme 20 in dem zweiten Schlauchabschnitt 14 geöffnet bzw. auch die Olive 19 abgebrochen. Nun fließt das Vollblut ausschließlich in den Vollblutbeutel 21, der vielfach auch Sammelbeutel genannt wird. Es ist auch möglich, dass nur die zunächst geschlossene Klemme 20 oder nur das Abbrechteil 18 im bzw. am zweiten Schlauchabschnitt angeordnet sind.

Bei der Verarbeitung zu einzelnen Präparaten wird das Vollblut im Vollblutbeutel 21 zunächst zentrifugiert. Im Anschluss werden die Komponenten: Erythrozyten in den Erythrozytenbeutel 22, Plasma in den Plasmabeutel 23 und Thrombozyten (verbleiben im Vollblutbeutel 21) getrennt. In Deutschland dürfen nach den Vorgaben der Bundesbehörde, dem Paul-Ehrlich-Institut, nur noch leukozytendepletierte Blutprodukte oder Produkte mit sehr geringem Leukozytenanteil in Verkehr gebracht werden. Die Leukozytendepletion bedeutet die Entfernung der Leukozyten aus den Präparaten. In der Regel werden wie beispielhaft in Fig. 2 dargestellt die weißen Blutkörperchen mithilfe eines Filters 25 ausdem Erythrozytenkonzentrat entfernt, sodass der Leukozytengehalt pro Einheit Präparat nach der Filtration weniger als 10⁻⁶ Leukozyten im Beutel 24 für das Leukozyten depletierte Erythrozytenkonzentrat beträgt. Als Filtermedien können oberflächenmodifizierte Polyesterfasern zum Einsatz kommen. Die Filtration erfolgt durch Adsorption der Leukozyten. Der Beutel 24 für das Leukozyten-abgereicherte Erythrozytenkonzentrat ist mit einer Additivlösung versehen.

## Patentansprüche

1. Blutbeutelsystem umfassend ein Predonationbeutelsystem (1) und zumindest einen Vollblutbeutel (21), wobei das Predonationbeutelsystem (1) zumindest umfasst:
einen Predonationbeutel (2), einen Venenpunktionsadapter (16) und eine Blutentnahmeleitung (4) mit
a) einem gegenüber der Blutentnahmeleitung (4) verschließbaren ersten Schlauchabschnitt (6) als Zufuhr von der Blutentnahmeleitung (4) in den Predonationbeutel (2) und
b) einen zweiten Schlauchabschnitt (14) als Zufuhr von der Blutentnahmeleitung (4) in den Vollblutbeutel (21),
wobei der Predonationbeutel (2) oder der verschließbare erste Schlauchabschnitt (6) oder beide ein erstes Antikoagulans für Vollblut umfasst/umfassen und das erste Antikoagulans EDTA ist; wobei der Predonationbeutel (2) oder der verschließbare erste Schlauchabschnitt (6) einen Adapter (11) zur Befüllung von einem oder mehreren Probenbehältern aufweist; und
wobei sich im Vollblutbeutel (21) ein von dem ersten Antikoagulans verschiedenes zweites Antikoagulans befindet, dass kein EDTA ist und CPD, CPDA1 und/oder CP2D ist.

2. Blutbeutelsystem nach Anspruch 1, wobei das erste Antikoagulans K2-EDTA oder K3-EDTA oder beides ist.

3. Blutbeutelsystem nach einem oder mehreren der vorherigen Patentansprüche, wobei das erste Antikoagulans
a) als Feststoff auf die innere Oberfläche des verschließbaren ersten Schlauchabschnitts oder eines Röhrchens, das in den verschließbaren ersten Schlauchabschnitt eingesetzt ist, aufgebracht ist und/oder
b) als Feststoff auf die innere Oberfläche des Predonationbeutels (2) aufgebracht ist.

4. Blutbeutelsystem nach Patentanspruch 1 oder 2, wobei das erste Antikoagulans in Wasser gelöst in den Predonationbeutel (2) eingebracht ist.

5. Blutbeutelsystem nach einem oder mehreren der vorherigen Patentansprüche, wobei der Predonationbeutel (2) ein Volumen von 10 bis 50 mL, insbesondere 25 bis 35 mL, aufweist.

6. Blutbeutelsystem nach einem oder mehreren der vorherigen Patentansprüche, wobei das erste Antikoagulans in einer Menge eingebracht wird, dass sich eine Konzentration im Vollblut des Predonationbeutel (2) von größer gleich 1,2 mg/mL, und vorzugsweise 1,4 bis 1,8 mg/mL ergibt.

7. Blutbeutelsystem nach einem oder mehreren der Patentansprüche 1, 2, 5 oder 6, wobei das erste Antikoagulans als Pille oder Tablette in den Predonationbeutel (2) oder den verschließbaren ersten Schlauchabschnitt eingebracht ist, insbesondere in einer Pillenkammer (8).

8. Blutbeutelsystem nach einem oder mehreren der vorherigen Patentansprüche, wobei der verschließbare erste Schlauchabschnitt und der Predonationbeutel (2) als totes Ende ausgebildet sind.

9. Blutbeutelsystem nach einem oder mehreren der vorherigen Patentansprüche, wobei der verschließbare erste Schlauchabschnitt einen zunächst offenen und später verschließbaren Verschluss (7) aufweist, insbesondere später irreversibel verschließbar.

10. Blutbeutelsystem nach einem oder mehreren der vorherigen Patentansprüche, wobei der erste Schlauchabschnitt (6) ein Abbrechteil mit Sollbruchstelle im Inneren des ersten Schlauchabschnitts (6) aufweist, das mechanisch abgebrochen wird und dadurch den Durchfluss in den Predonationbeutel über den verschließbaren ersten Schlauchabschnitt freigibt und/oder
wobei der zweite Schlauchabschnitt (14) ein Abbrechteil (18) mit Sollbruchstelle im Inneren des zweiten Schlauchabschnitts (14) aufweist, das mechanisch abgebrochen wird und dadurch den Durchfluss in den Vollblutbeutel (21) über den ersten Schlauchabschnitt freigibt.

11. Blutbeutelsystem nach einem oder mehreren der vorherigen Patentansprüche, wobei der verschließbare erste Schlauchabschnitt einen verschließbaren Verschluss (7) aufweist, wobei das erste Antikoagulanz vorzugsweise ausschließlich im verschließbaren ersten Schlauchabschnitt hinter dem Verschluss und/oder ausschließlich im Predonationbeutel (2) angeordnet ist.

12. Blutbeutelsystem nach einem oder mehreren der vorherigen Patentansprüche, wobei sich im zweiten Schlauchabschnitt (14) eine reversible Klemme befindet zum Öffnen und wieder Schließen und ggf. wieder Öffnen des Zuflusses in den Vollblutbeutel (21).

13. Blutbeutelsystem nach einem oder mehreren der Patentansprüche 1 bis 11, wobei sich im zweiten Schlauchabschnitt (14) eine Klemme befindet, die zunächst geschlossen ist, sich öffnen lässt und danach reversibel oder irreversibel wieder schließt.

14. Verfahren zur zentralen Befüllung von einem oder mehreren Probenbehältern mittels eines Blutbeutelsystems nach einem oder mehreren der Patentansprüche 1 bis 13, wobei das mit erstem Antikoagulans versehene Vollblut oder ein Teil davon aus dem Predonationbeutel (2) nach Transport in ein Labor mittels des Adapters (11) in eine oder mehrere Probenbehälter gefüllt wird, vorzugsweise automatisiert, und der Probenbehälter einer Analyse zugeführt wird oder die Probenbehälter jeweils unterschiedlichen Analysen zugeführt werden.

15. Verfahren nach Patentanspruch 14, wobei die Anzahl der Probenbehälter pro Predonationbeutel (2) in Abhängigkeit von dem Spender und einem Abgleich mit einem Eintrag in einer Datenbank für den Spender bestimmt werden und die Probenbehälter und deren Anzahl im Hinblick auf die mittels des Eintrags in die Datenbank ausgewählten Tests gekennzeichnet werden und vorzugsweise Probenbehälter unterschiedlicher Spender, wobei jedem Spender nur ein definiertes Blutbeutelsystem zuzuordnen ist, automatisiert aufgrund der Kennzeichnung sortiert und im Hinblick auf gleiche Tests oder Abfolgen von gleichen Tests für die unterschiedlichen Spender zusammengestellt werden.

## Claims

1. Blood bag system comprising a predonation bag system (1) and at least one whole blood bag (21), wherein the predonation bag system (1) comprises at least:
a predonation bag (2), a venipuncture adapter (16) and a blood collection line (4) with
a) a first tube section (6) which is sealable with respect to the blood collection line (4) as a feed from the blood collection line (4) into the predonation bag (2) and
b) a second tube section (14) as a feed from the blood collection line (4) into the whole blood bag (21),
wherein the predonation bag (2) or the sealable first tube section (6) or both comprises/comprise a first anticoagulant for whole blood and the first anticoagulant is EDTA; wherein the predonation bag (2) or the sealable first tube section (6) comprises an adapter (11) for filling of one or more sample containers; and
wherein in the whole blood bag (21) a second anticoagulant is contained, which is different from the first anticoagulant and which is not EDTA and is CPD, CPDA1 and/or CP2D.

2. Blood bag system according to claim 1, wherein the first anticoagulant is K2-EDTA or K3-EDTA or both.

3. Blood bag system according to one or more of the preceding claims, wherein the first anticoagulant
a) is applied as a solid to the inner surface of the sealable first tube section or of a small tube inserted into the sealable first tube section and/or
b) is applied as a solid to the inner surface of the predonation bag (2).

4. Blood bag system according to claim 1 or 2, wherein the first anticoagulant is introduced into the predonation bag (2) dissolved in water.

5. Blood bag system according to one or more of the preceding claims, wherein the predonation bag (2) has a volume of 10 to 50 mL, in particular 25 to 35 mL.

6. Blood bag system according to one or more of the preceding claims, wherein the first anticoagulant is introduced in an amount such that a concentration in the whole blood of the predonation bag (2) of greater than or equal to 1.2 mg/mL, and preferably 1.4 to 1.8 mg/mL is obtained.

7. Blood bag system according to one or more of the claims 1, 2, 5 or 6, wherein the first anticoagulant is introduced as a pill or tablet into the predonation bag (2) or the sealable first tube section, in particular in a pill chamber (8).

8. Blood bag system according to one or more of the preceding claims, wherein the sealable first tube section and the predonation bag (2) are formed as a dead end.

9. Blood bag system according to one or more of the preceding claims, wherein the sealable first tube section has an initially open and later sealable closure (7), in particular later irreversibly closeable.

10. Blood bag system according to one or more of the preceding claims, wherein the first tube section (6) has a break-off part with a predetermined breaking point inside the first tube section (6), which is mechanically broken off and thereby releases the flow into the predonation bag via the sealable first tube section and/or wherein the second tube section (14) has a break-off part (18) with a predetermined breaking point inside the second tube section (14), which is mechanically broken off and thereby releases the flow into the whole blood bag (21) via the first tube section.

11. Blood bag system according to one or more of the preceding claims, wherein the sealable first tube section comprises a sealable closure (7), wherein the first anticoagulant is preferably arranged exclusively in the sealable first tube section behind the closure and/or exclusively in the predonation bag (2).

12. Blood bag system according to one or more of the preceding claims, wherein a reversible clamp is located in the second tube section (14) for opening and closing again and, optionally, reopening the inflow into the whole blood bag (21).

13. Blood bag system according to one or more of claims 1 to 11, wherein a clamp is located in the second tube section (14), which is initially closed, can be opened and then closes again reversibly or irreversibly.

14. Method for central filling of one or more sample containers by means of a blood bag system according to one or more of claims 1 to 13, wherein the whole blood provided with the first anticoagulant or a part thereof from the predonation bag (2) after transportation to a laboratory is filled by means of the adapter (11) into one or more sample containers, preferably automatically, and the sample container is supplied to an analysis or the sample containers are each supplied to different analyses.

15. Method according to claim 14, wherein the number of sample containers per predonation bag (2) is determined as a function of the donor and a comparison with an entry in a database for the donor and the sample containers and their number are labeled with regard to the tests selected by means of the entry in the database and preferably sample containers of different donors, wherein only one defined blood bag system is assigned to each donor, are automatically sorted on the basis of the labeling and are compiled with regard to identical tests or sequences of identical tests for the different donors.

## Revendications

1. - Système de poche de sang comportant au moins un système de poche de pré-transfusion (1) et au moins une poche de sang total (21), le système de poche de pré-transfusion comportant au moins : une poche de pré-transfusion (2), un adaptateur de ponction veineuse (16) et un tube de prélèvement sanguin (4) avec
a) une première section de tube (6) pouvant être fermée par rapport au tube de prélèvement sanguin (4) comme alimentation du tube de prélèvement sanguin (4) dans la poche de pré-transfusion (2), et
b) une seconde section de tube (14) comme alimentation du tube de prélèvement sanguin (4) dans la poche de sang total (21),
la poche de pré-transfusion (2) ou la première section de tube (6) pouvant être fermée ou les deux comprenant un premier anticoagulant pour le sang total, et le premier anticoagulant étant l'EDTA ; la poche de pré-transfusion (2) ou la première section de tube (6) pouvant être fermée présentant un adaptateur (11) pour le remplissage d'un ou plusieurs récipients d'échantillons ; et
où, dans la poche de sang total (21), se trouve un second anticoagulant différent du premier anticoagulant, qui n'est pas l'EDTA et qui est CPD, CPDA1 et/ou CP2D.

2. - Système de poche de sang selon la revendication 1, dans lequel le premier anticoagulant est du K2-EDTA ou du K3-EDTA ou les deux.

3. - Système de poche de sang selon l'une ou plusieurs des revendications précédentes, dans lequel le premier anticoagulant
a) est appliqué sous forme solide sur la surface interne de la première section de tube pouvant être fermée ou d'un petit tube qui est introduit dans la première section de tube pouvant être fermée, et/ou
b) est appliqué sous forme solide sur la surface interne de la poche de pré-transfusion (2).

4. - Système de poche de sang selon l'une des revendications 1 et 2, dans lequel le premier anticoagulant est introduit dans la poche de pré-transfusion (2) sous forme dissoute dans l'eau.

5. - Système de poche de sang selon l'une ou plusieurs des revendications précédentes, dans lequel la poche de pré-transfusion (2) a un volume de 10 à 50 mL, en particulier de 25 à 35 mL.

6. - Système de poche de sang selon l'une ou plusieurs des revendications précédentes, dans lequel le premier anticoagulant est introduit dans une quantité telle qu'il en résulte une concentration dans le sang total de la poche de pré-transfusion (2) supérieure ou égale à 1,2 mg/mL, et de préférence de 1,4 à 1,8 mg/mL.

7. - Système de poche de sang selon l'une ou plusieurs des revendications 1, 2, 5 et 6, dans lequel le premier anticoagulant est introduit sous forme de pilule ou de comprimé dans la poche de pré-transfusion (2) ou dans la première section de tube pouvant être fermée, en particulier dans un compartiment à pilules (8).

8. -Système de poche de sang selon l'une ou plusieurs des revendications précédentes, dans lequel la première section de tube pouvant être fermée et la poche de pré-transfusion (2) sont conçues comme une extrémité morte.

9. - Système de poche de sang selon l'une ou plusieurs des revendications précédentes, dans lequel la première section de tube pouvant être fermée présente une fermeture (7) d'abord ouverte et ensuite pouvant être fermée, en particulier pouvant être ensuite fermée de façon irréversible.

10. - Système de poche de sang selon l'une ou plusieurs des revendications précédentes, dans lequel la première section de tube (6) présente une partie cassable avec un point de rupture prédéterminé à l'intérieur de la première section de tube (6), qui est cassée mécaniquement et libère ainsi le débit dans la poche de pré-transfusion par l'intermédiaire de la première section de tube pouvant être fermée, et/ou
dans lequel la seconde section de tube (14) présente une partie cassable (18) avec un point de rupture à l'intérieur de la seconde section de tube (14), qui est cassée mécaniquement et libère ainsi le débit dans la poche de sang total (21) par l'intermédiaire de la première section de tube.

11. - Système de poche de sang selon l'une ou plusieurs des revendications précédentes, dans lequel la première section de tube pouvant être fermée présente une fermeture (7) pouvant être fermée, le premier anticoagulant étant de préférence disposé exclusivement dans la première section de tube pouvant être fermée derrière la fermeture et/ou exclusivement dans la poche de pré-transfusion (2).

12. - Système de poche de sang selon l'une ou plusieurs des revendications précédentes, dans lequel se trouve dans la seconde section de tube (14) une pince réversible pour ouvrir et refermer et, le cas échéant, ouvrir à nouveau l'écoulement dans la poche de sang total (21).

13. - Système de poche de sang selon l'une ou plusieurs des revendications 1 à 11, dans lequel se trouve dans la seconde section de tube (14) une pince qui est d'abord fermée, peut être ouverte, puis se referme de manière réversible ou irréversible.

14. - Procédé de remplissage central d'un ou plusieurs récipients d'échantillons à l'aide d'un système de poche de sang selon l'une ou plusieurs des revendications 1 à 13, dans lequel le sang total additionné d'un premier anticoagulant ou une partie de celui-ci est introduit de la poche de pré-transfusion (2), après transport dans un laboratoire, au moyen d'un adaptateur (11) dans un ou plusieurs récipients d'échantillons, de préférence de manière automatisée, et le récipient d'échantillon est soumis à une analyse ou les récipients d'échantillons sont soumis chacun à différentes analyses.

15. - Procédé selon la revendication 14, dans lequel le nombre des récipients d'échantillons par poche de pré-transfusion (2) est déterminé en fonction du donneur et d'une comparaison avec une entrée dans une base de données pour le donneur, et les récipients d'échantillons et leur nombre sont marqués en vue des tests sélectionnés au moyen de l'entrée dans la base de données, et de préférence, les récipients d'échantillons de différents donneurs, chaque donneur ne pouvant être associé qu'à un seul système de poches de sang défini, sont triés automatiquement sur la base du marquage et regroupés en fonction des mêmes tests ou des séries de mêmes tests pour les différents donneurs.
